# EUROPÄISCHE PATENTANMELDUNG

(11) **EP 4 074 784 A1**
(43) Veröffentlichungstag der Anmeldung: **19.10.2022**
(21) Anmeldenummer: 21167984.0
(22) Anmeldetag: 13.04.2021
(51) Int. Cl.: C09D 5/14, A61L 2/08, A61L 2/10, C09D 5/22, C09K 11/77

(54) **ZUSAMMENSETZUNG ZUR HERSTELLUNG VON BESCHICHTUNGEN MIT VERBESSERTEN LEUCHTSTOFFEN**

(71) Anmelder: Evonik Operations GmbH, 45128 Essen (DE)
(72) Erfinder: SCHULTE, Simone, 45133 Essen (DE); HALLACK, Markus, 46514 Schermbeck (DE); JANKE, Christina, 45327 Essen (DE); KRUSENBAUM, Sabine, 45134 Essen (DE); WOLFF, Bärbel, 47441 Moers (DE); JÜSTEL, Thomas, 58455 Witten (DE); FISCHER, Stefan, 59494 Soest (DE); SCHRÖDER, Franziska, 48565 Steinfurt (DE); REETZ, Sven, 48565 Steinfurt (DE); HUTH, Michael, 63477 Maintal (DE)
(74) Vertreter: Evonik Patent Association

(57) **Zusammenfassung**

Die Erfindung betrifft eine härtbare Zusammensetzung zur Herstellung von Beschichtungen mit anti-mikrobieller Eigenschaft enthaltend
- mindestens ein filmbildendes Polymer,
- optional mindestens ein Additiv,
- optional mindestens einen Härter,
- mindestens einen up-conversion Leuchtstoff der allgemeinen Formel (I)

A_{1-x-y-z}B*_{y}B₂SiO₄:Ln¹ₓ,Ln²_{z}, I

mit
x = 0,0001 - 0,0500;
z = 0,0000 oder z = 0,0001 bis 0,3000 mit der Maßgabe, dass gilt: y = x + z;
A ausgewählt aus der Gruppe bestehend aus Mg, Ca, Sr und Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
Ln¹ ausgewählt aus der Gruppe bestehend aus Praseodym (Pr), Erbium (Er) und Neodym (Nd);
Ln² ausgewählt aus Gadolinium (Gd),
wobei der Leuchtstoff mit mindestens einem halogenhaltigen Flussmittel hergestellt wurde.

## Beschreibung

Die Erfindung betrifft eine härtbare Zusammensetzung zur Herstellung von Beschichtungen mit anti-mikrobieller Eigenschaft, deren Verwendung und daraus hergestellte Beschichtungen und Gegenstände, die damit beschichtet sind.

Tagtäglich sind Menschen Millionen von Mikroorganismen wie Bakterien, Pilzen und Viren ausgesetzt. Viele dieser Mikroorganismen sind nützlich bzw. sogar notwendig. Dennoch gibt es neben den harmloseren Vertretern auch krankheitsverursachende oder sogar tödliche Bakterien, Pilze und Viren.

Durch den täglichen Umgang mit anderen Menschen und den Kontakt mit Gegenständen, die andere benutzt haben, können Mikroorganismen übertragen werden. Die antimikrobielle Ausstattung von Oberflächen erfolgt insbesondere in hygienesensiblen Bereichen. Anwendungsbereiche sind vor allem Oberflächen von medizintechnischen Geräten und Bedarfsgegenstände in Krankenhäusern sowie in Einrichtungen des ambulanten Gesundheits- und Sozialwesens. Hinzu kommen Oberflächen im öffentlichen Raum, im Lebensmittelsektor und in der Tierhaltung. Die Verbreitung von pathogenen Mikroorganismen stellt heute ein großes Problem im Pflegebereich und in der Medizin dar, und auch überall dort, wo viele Menschen auf engem Raum verkehren. Ein besonders Risiko stellt gegenwärtig auch das vermehrte Aufkommen sogenannter multiresistenter Keime dar, die unempfindlich gegen übliche Antibiotika geworden sind.

Um das Risiko der Verbreitung von pathogenen Erregern über Berührungsoberflächen zu verringern, werden, neben Maßnahmen zur Standardhygiene, antimikrobielle Technologien und Werkstoffe genutzt. Chemische Substanzen oder die Verwendung physikalischer Verfahren können den Vermehrungsprozess von Mikroorganismen kritisch beeinflussen. Zu den physikalischen Methoden zählen z. B. Hitze, Kälte, Strahlung oder Ultraschall, etc. Bei den chemischen Methoden sind Halogene, Metallionen, organische Verbindungen und Farbstoffe, giftige Gase, etc. bekannt.

Obwohl in den meisten Fällen chemische und physikalische Methoden außerordentlich effektiv bei der Zerstörung von Mikroorganismen sind, so haben sie doch nur einen kurzzeitigen Effekt, chemische Methoden fördern die Entstehung von Resistenzen und sind unter Umständen für manche Anwendungen nicht geeignet, da sie zur Zerstörung der zu schützenden Oberflächen führen. Den größten Nachteil stellt allerdings, gerade bei chemischen, organischen Substanzen, die Gefährlichkeit bzw. Giftigkeit für den Menschen dar. Bestimmte Substanzen, wie z. B. Formaldehyd, welches viele Jahre als Desinfektionsmittel Anwendung fand, stehen inzwischen im Verdacht, Krebs zu verursachen oder extrem umweltschädlich zu sein.

Oberflächen mit antimikrobieller Wirkung könnten einen entscheidenden Beitrag zur Lösung dieser Probleme leisten. Die heute gängigen Verfahren zur Erzeugung solcher antimikrobiellen Eigenschaften verwenden überwiegend in das Material eingearbeitete Wirkstoffe wie z.B. Silberpartikel, Kupferpartikel, deren Metalloxide oder quaternäre Ammoniumverbindungen. Dabei werden die antimikrobiell wirkenden Metalle, Metalloxide oder Metalloxidgemische häufig zu Nanoteilchen verarbeitet und dann Farben, Lacken oder Polymerwerkstoffen zugemischt. Der breite Einsatz von Metallpartikeln ist in Frage zu stellen, da die langfristige Wirkung dieses Schwermetalls auf Menschen und Umwelt kaum abzuschätzen ist.

Beispielsweise offenbart die WO 2019/197076 Partikel, die mit einer Schicht ausgestattet sind, die sowohl Antimon-Zinnoxid als auch Manganoxid enthält. Dem Fachmann ist bekannt, dass die antimikrobiellen Oberflächen aufgrund des elektrochemischen Verhaltens von Metallen erzeugt werden, die bei Anwesenheit von Feuchtigkeit mikrogalvanische Zellen und durch die mikroelektrischen Felder keimabtötende Wirkung entfalten.

Es ist ebenso bekannt, UV-Strahlung in der Medizin oder in der Hygiene einzusetzen, um beispielsweise Wasser, Gase oder Oberflächen zu desinfizieren. So wird in der Trinkwasseraufbereitung seit langem UV-Strahlung zur Reduzierung der Anzahl von fakultativ krankheitserregenden Mikroorganismen im Wasser eingesetzt. Dabei wird vorzugsweise UV-C-Strahlung im Wellenlängenbereich zwischen 200 nm und 280 nm eingesetzt. Der Einsatz elektromagnetischer Strahlung mit unterschiedlichen Wellenlängen sollte die unterschiedliche Absorption der verschiedenen Proteine, den in Mikroorganismen, Geweben oder Zellen enthaltenden Amino-/Nukleinsäuren (z.B. DNA oder RNA) sowie Peptidbindungen zwischen den einzelnen Säuren berücksichtigen. So absorbiert DNA/RNA elektromagnetische Strahlung innerhalb des Wellenlängenbereichs zwischen 200 nm und 300 nm gut und zwischen 250 nm und 280 nm besonders gut, so dass diese Strahlung zur Inaktivierung von DNA/RNA besonders geeignet ist. Es können also krankheitserregende Mikroorganismen (Viren, Bakterien, Hefen, Schimmelpilze u.a.) mit einer solchen Bestrahlung inaktiviert werden. Je nach Dauer und Intensität der Bestrahlung kann die Struktur von DNA bzw. RNA zerstört werden. Somit werden stoffwechselaktive Zellen inaktiviert und/oder deren Vermehrungsvermögen kann beseitigt werden. Vorteilhaft bei der Bestrahlung mit UV-Strahlung ist, dass die Mikroorganismen keine Resistenz dagegen entwickeln können. Diese physikalischen Methoden erfordern allerdings spezielle Apparaturen und müssen in der Regel von geschultem Personal regelmäßig wiederholt werden, was einen breiten Einsatz dieser Methoden erschwert.

Des Weiteren ist es neben einer direkten Bestrahlung mit elektromagnetischer Strahlung aus dem Wellenlängenbereich der UV-Strahlung auch bekannt, den Effekt der so genannten up-conversion auszunutzen. Dabei werden Leuchtstoffpartikel eingesetzt, mit denen elektromagnetische Strahlung mit Wellenlängen oberhalb des UV-Strahlung, insbesondere sichtbares Licht oder Infrarotstrahlung, in elektromagnetische Strahlung mit geringerer Wellenlänge gewandelt werden kann, so dass die Emission von in gewünschter Form wirkender Strahlung von den einzelnen Leuchtstoffpartikeln erreicht werden kann.

DE 10 2015 102 427 betrifft einen elektromagnetische Strahlung im Wellenlängenbereich des UV-Lichts emittierenden Körper. Bei dem Körper sind in einem oberflächennahen Bereich innerhalb des Werkstoffs, aus dem der Körper gebildet ist oder einer Beschichtung auf dem Körper, Leuchtstoffpartikel eingebettet. Hierbei wird nur allgemein angegeben, dass die Leuchtstoffpartikel einer auf dem Werkstoff auszubildenden Beschichtung direkt bei der Verarbeitung zugegeben werden, dabei sollte der jeweilige Werkstoff eine geeignete Konsistenz bzw. Viskosität aufweisen. Hinsichtlich geeigneter Polymere und Additive schweigt DE 10 2015 102 427.

US 2009/0130169 A1 bzw. WO 2009/064845 A2 beschreibt Leuchtstoffe, die in Polyvinylchloride, Acrylbutadiene, Polyolefine, Polycarbonate, Polystyrole oder Nylon eingebracht werden können, welche durch die up-conversion Eigenschaft der Leuchtstoffe pathogene Mikroorganismen abtöten. Es handelt sich hierbei um Leuchtstoffe, die bei einer Temperatur von 1800 - 2900 °C hergestellt werden. Die US 2009/0130169 A1 bzw. WO 2009/064845 A2 offenbart zwar eine Zusammensetzung enthaltend besagter Leuchtstoffe mit behaupteter antimikrobieller Wirkung, zeigt jedoch weder einen Nachweis der up-conversion Eigenschaft noch mikrobiologischer Untersuchungen. Das darin offenbarte Verfahren führt zu keinem Leuchtstoff, der eine up-conversion Eigenschaft aufweist, sondern zu einem amorphen und glasartigen Produkt.

Darüber hinaus schweigt die US 2009/0130169 A1 bzw. WO 2009/064845 A2 über die Verträglichkeit der Komponente in der Beschichtungszusammensetzung oder die Eigenschaften der Beschichtungsoberflächen, wie etwa die Lackoberflächen. Das Erscheinungsbild von Beschichtungsoberflächen spielen jedoch beim Anwender eine besondere Rolle.

Die Anforderungen an Lacke und Farben sind vielfältig. Grundsätzlich haben Lack- bzw. Farbbeschichtungen zwei Aufgaben bzw. Funktionen, die schützende und die dekorative Funktion. Sollten im Weiteren nur der Begriff "Lackbeschichtung" aufgeführt sein, so sind beide Arten der Beschichtung gemeint. Sie verschönern, schützen und bewahren Materialien wie Holz, Metall oder Kunststoff. Demnach werden auf der einen Seite brillante und glatte Lackschichten gefordert, auf der anderen Seite eine geschlossene Lackschicht zur Sicherstellung der chemischen und mechanischen Beständigkeit, eine gewisse Gleitfähigkeit der Lacke oder eine besondere Haptik.

Im Gegensatz zu der WO 2009/064845 A2 konnten die noch nicht offengelegten Patentanmeldungen EP 19202910.6 und PCT/EP2020/077798 Leuchtstoffe und deren Herstellung offenbaren, die eine up-conversion aufweisen. Solche Leuchtstoffe können bei Bestrahlung mit elektromagnetischer Strahlung mit geringerer Energie und höherer Wellenlänge im Bereich von 2000 nm bis 400 nm, insbesondere im Bereich von 800 nm bis 400 nm, elektromagnetische Strahlung mit höherer Energie bzw. kürzerer Wellenlänge im Bereich von 400 nm bis 100 nm, bevorzugt im Bereich von 300 nm bis 200 nm emittieren, so dass sie geeignet sind, als anti-mikrobielle Leuchtstoffe in Lackbeschichtungen eingesetzt zu werden.

So beschreibt die noch nicht offengelegte Europäische Patentanmeldungen EP 21157055.1 eine Zusammensetzung enthalten mindestens ein filmbildendes Polymer, mindestens einen up-conversion Leuchtstoff gemäß der Lehre der EP 19202910.6 und PCT/EP2020/077798, optional mindestens ein Additiv und optional mindestens einen Härter. Es konnte gezeigt werden, dass Lackbeschichtungen enthaltend dieser Leuchtstoffe antimikrobiell wirken und ohne dass die übrigen Eigenschaften, insbesondere die Lagerstabilität, signifikant beeinträchtigt werden.

Es wurde jedoch auch festgestellt, dass die Leuchtstoffe, hergestellt nach einem Verfahren gemäß der EP 19202910.6 und PCT/EP2020/077798, eine inhomogene Partikelgrößenverteilung aufweisen, was bei deren Einarbeitung in einer Lackmatrix eine besondere Herausforderung darstellt. Auch wenn die Lehre der EP 21157055.1 zu anti-mikrobiellen wirkenden Lackbeschichtungen führt, so wäre es weiterhin wünschenswert, wenn die Intensität der Emission der Leuchtstoffe erhöht werden könnte.

Dementsprechend stellt sich die Erfindung die Aufgabe, eine härtbare Zusammensetzung der eingangs genannten Art zur Verfügung zu stellen, mit der Beschichtungen hergestellt werden können, bei denen ein Schutz gegen Mikroorganismen bereitgestellt wird, wobei die up-conversion Leuchtstoffe eingesetzt werden, die eine homogene Partikelgrößenverteilung aufweisen und zudem eine höhere Intensität der Emission aufzeigen.

Basierend auf die Lehre der Europäischen Patentanmeldungen EP 19202910.6, PCT/EP2020/077798 und EP 21157055.1 schlägt die vorliegende Erfindung vor, eine Härtbare Zusammensetzung zur Herstellung von Beschichtungen mit anti-mikrobieller Eigenschaft enthaltend
- mindestens ein filmbildendes Polymer,
- optional mindestens ein Additiv,
- optional mindestens einen Härter,
- mindestens einen up-conversion Leuchtstoff der allgemeinen Formel (I)

   A_{1-x-y-z}B*_{y}B₂SiO₄:Ln¹ₓLn²_{z}, I

   mit
   x = 0,0001 - 0,0500;
   z = 0,0000 oder z = 0,0001 bis 0,3000 mit der Maßgabe, dass gilt: y = x + z;
   A ausgewählt aus der Gruppe bestehend aus Mg, Ca, Sr und Ba;
   B ausgewählt aus der Gruppe bestehend aus Li, Na, K, Rb und Cs;
   B* ausgewählt aus der Gruppe bestehend aus Li, Na und K, wobei B gleich B* oder B ungleich B* ist, bevorzugt B und B* sind ungleich;
   Ln¹ ausgewählt aus der Gruppe bestehend aus Praseodym (Pr), Erbium (Er) und Neodym (Nd);
   Ln² ausgewählt aus Gadolinium (Gd),
   wobei der Leuchtstoff mit mindestens einem halogenhaltigen Flussmittel hergestellt wurde.

Aus dem Stand der Technik sind zahlreiche Flussmittel aller Art, wie Halogenide, Carbonate, Sulfate, Oxide und Borate von jeweils, wo zutreffend, Ammonium, Lithium, Natrium, Kalium, Rubidium, Cäsium, Magnesium, Calcium, Strontium, Barium, Blei, Lanthan, Lutetium, Aluminium, Bismut sowie Borsäure, dem Fachmann bekannt. Auch bekannt sind deren Einsatzgebiete im Bereich der Metallurgie, um beispielsweise das Kristallwachstum zu beschleunigen oder die Bildung von Fremdphasen zu unterbinden.

Daher war es auch eine Aufgabe der Erfindung gewesen, ein geeignetes Flussmittel auszuwählen, damit die gewünschten Eigenschaften erzielt werden konnten. Das Auffinden geeigneter Flussmittel war daher von besonderer Bedeutung, so dass auch hier bereits der Beitrag zur Erfindungshöhe gegeben ist.

Völlig überraschend konnte festgestellt werden, dass die Herstellung der up-conversion Leuchtstoffe in Gegenwart von mindestens einem halogenhaltigen Flussmittel zu up-conversion Leuchtstoffe mit homogener Partikelgrößenverteilung sowie erhöhter Intensität der Emission bzw. höherer Quantenausbeute führen, im Vergleich zu Leuchtstoffen ohne Zusatz eines Flussmittels oder mit einem anderen Flussmittel.

Flussmittel wird im englischsprachigen Raum als "flux" übersetzt; daher ist auch der Begriff Fluxmittel bekannt. Die Behandlung mit Flussmittel wird auch fluxen genannt bzw. das Produkt wurde gefluxt.

Es konnte in den Beispielen gezeigt werden, dass die Partikelgrößenverteilung des erfindungsgemäßen gefluxten Leuchtstoffes einer Gauß'schen Verteilung ähnelt, was auf eine Homogenität der Partikelgröße hindeutet, sodass vorteilhafterweise deren Einarbeitung in einer Lackmatrix wesentlich leichter durchgeführt werden kann. Es wird angenommen, dass dadurch die Lackeigenschaften, wie etwa das Erscheinungsbild der Lackoberfläche, beispielsweise der Glanz, Haptik und Touch, verbessert wurden.

Auch konnte die Intensität der Emission der up-conversion Leuchtstoffe durch eine einfache technische Durchführung der Synthese erzielt werden.

Somit ist auch ein weiterer Gegenstand der Erfindung ein Verfahren zur Herstellung dieser up-conversion Leuchtstoffe sowie damit erhältliche up-conversion Leuchtstoffe.

Vorzugsweise wird als halogenhaltiges Flussmittel mindestens ein Stoff aus der Gruppe der Ammoniumhalogenide, Alkalimetallhalogenide, Erdalkalimetallhalogenide und Lanthanoidhalogenide eingesetzt. Halogenide dieser Gruppen haben überraschend gezeigt, dass damit hergestellte up-conversion Leuchtstoffe eine höhere Emission-Intensität aufweisen als mit anderen Flussmitteln.

Vorzugsweise handelt es sich bei den Halogeniden um Fluoride oder Chloride.

Bevorzugt handelt es sich bei den Alkalimetallen um Kalium, Natrium oder Lithium.

Bevorzugt handelt es sich bei dem Lanthanoid um Praseodym.

Bevorzugt handelt es sich bei den Erdalkalimetallen um Calcium oder Strontium.

Vorzugsweise ist der Leuchtstoff mit Praseodym dotiert, der in der erfindungsgemäßen Zusammensetzung verwendet wird.

Bevorzugt für die erfindungsgemäße Zusammensetzung ist der Leuchtstoff mit Praseodym dotiert und mit Gadolinium co-dotiert.

Es ist bevorzugt, dass der Leuchtstoff zumindest partiell kristallin ist. Es ist also bevorzugt, dass der Leuchtstoff partiell oder vollständig kristallin ist. Vorzugsweise ist der Leuchtstoff also zumindest nicht vollständig amorph. Es ist daher bevorzugt, dass der Leuchtstoff keine amorph erstarrte Schmelze (Glas) ist.

Vorzugsweise ist der Leuchtstoff eine erstarrte Schmelze aus kristallinen Silikaten oder aus kristallinen Silikaten, dotiert mit Lanthanoid-Ionen, umfassend mindestens einen Alkalimetallion und mindestens einen Erdalkalimetallion.

Bevorzugt für die erfindungsgemäße Zusammensetzung ist der Leuchtstoff ausgewählt aus Verbindungen der allgemeinen Formel (la)

A_{1-x-y-z}B*_{y}B₂SiO₄:Prₓ,Gd_{z}, **(la)**

mit A ausgewählt aus der Gruppe bestehend aus Mg, Ca, Sr, Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
x = 0,0001 - 0,0500;
z = 0,0000 oder z = 0,0001 bis 0,3000 mit der Maßgabe, dass gilt: y = x + z.

B* dient dabei zum Ladungsausgleich der Silikatanionen.

A kann dabei für ein einziges Element aus der Gruppe bestehend aus Mg, Ca, Sr und Ba stehen, oder auch für eine Kombination von zwei oder mehr Elementen dieser Gruppe, also z.B. A = (Mgₐ Caₐ₂Srₐ₃Baₐ₄) mit 0 ≤ a1 ≤ 1, 0 ≤ a2 ≤ 1, 0 ≤ a3 ≤ 1 0≤ a4 ≤ 1 und mit der Maßgabe, dass gilt: a1+a2+a3+a4 = 1. A kann also z.B. für (Ca_{0,9}Sr_{0,1}) stehen.

Vorzugsweise wird für die erfindungsgemäße Zusammensetzung der Leuchtstoff ausgewählt aus Verbindungen der allgemeinen Formel (II)

(Ca₁₋ₐSrₐ)_{1-2b}Ln_{b}Na_{b}Li₂SiO₄ **II**

wobei gilt:
Ln ist ausgewählt aus der Gruppe bestehend aus Praseodym, Gadolinium, Erbium, Neodym, vorzugsweise Praesodym;
a = 0,0000 bis 1,0000, vorzugsweise 0,0000 bis 0,1000, insbesondere 0,0000;
b = 0,0001 bis 1,0000, vorzugsweise 0,0001 bis 0,1000, insbesondere 0,0050 bis 0,0150.

Ln kann dabei für ein einziges Element aus der Gruppe bestehend aus Praseodym, Gadolinium, Erbium und Neodym stehen, oder auch für eine Kombination von zwei Elementen dieser Gruppe, also z.B. Ln = (Ln¹ₓLn²_{y}), wobei Ln¹ und Ln² ausgewählt sind der Gruppe bestehend aus Praseodym, Gadolinium, Erbium und Neodym, und wobei x und y wie für Formel (I) und (la) definiert sind.

Ln¹ dient der Dotierung. Für die Dotierung wird vorzugsweise Praesodym verwendet. Ln² dient der optionalen Co-Dotierung. Für die optionale Co-Dotierung wird vorzugsweise Gadolinium verwendet. Vorzugsweise ist der Leuchtstoff nicht co-dotiert, d.h. Ln steht für vorzugsweise für ein einziges Element aus der Gruppe bestehend aus Praseodym, Gadolinium, Erbium und Neodym.

Es ist noch bevorzugter, dass der Leuchtstoff ausgewählt ist aus Verbindungen der allgemeinen Formel (IIa)

Ca_{1-2b}Pr_{b}Na_{b}Li₂SiO₄ (IIa)

mit b = 0,0001 bis 1, vorzugsweise 0,0001 bis 0,1, insbesondere 0,005 bis 0,015.

Es ist ganz besonders bevorzugt, dass der Leuchtstoff Ca_{0,98}Pr_{0,01}Na_{0,01}Li₂SiO₄ ist.

Bevorzugt weist der erfindungsgemäße up-conversion Leuchtstoff ein Halogen, entsprechend dem Halogenid des Flussmittels, auf.

Bevorzugt handelt es sich bei dem Leuchtstoff um einen, der bei Bestrahlung mit elektromagnetischer Strahlung mit geringerer Energie und höherer Wellenlänge im Bereich von 2000 nm bis 400 nm, insbesondere im Bereich von 800 nm bis 400 nm, elektromagnetische Strahlung mit höherer Energie und kürzerer Wellenlänge im Bereich von 400 nm bis 100 nm, bevorzugt im Bereich von 300 nm bis 200 nm emittiert. Es ist ferner bevorzugt, dass dabei die Intensität des Emissionsmaximums der elektromagnetischen Strahlung mit höherer Energie und kürzerer Wellenlänge eine Intensität von mindestens 1 • 10³ counts/(mm^{2*}s), bevorzugt höher als 1 • 10⁴ counts/(mm^{2*}s), besonders bevorzugt höher als 1 • 10⁵ counts/(mm^{2*}s). Zur Bestimmung dieser Kenngrößen wird die Emission bevorzugt mittels eines Lasers, insbesondere eines Lasers mit einer Leistung von 75 mW bei 445 nm und/oder einer Leistung von 150 mW bei 488 nm angeregt.

Vorzugsweise weist der Leuchtstoff gemäß Formel (II) XRPD Signale im Bereich von 23° 2Θ to 27° 2Θ und von 34° 2Θ bis 39.5° 2Θ auf, wobei die Signale mittels der Bragg-Brentano Geometrie und der Cu-K_{α} Radiation bestimmt werden. Einzelheiten der Messmethode können aus der noch unveröffentlichten europäischen Patentanmeldungen EP 19202910.6 und PCT/EP2020/077798 entnommen werden.

Die bisher unveröffentlichten europäischen Patentanmeldungen EP 19202910.6 und PCT/EP2020/077798 widmen sich der Herstellung von Leuchtstoffen, insbesondere von Leuchtstoffen gemäß Formel (I), Formel (la) und Formel (II), ohne den Zusatz von Flussmitteln.

Ausgehend des darin beschriebenen Verfahrens umfasst das erfindungsgemäße Verfahren folgende Schritte:
- i) Bereitstellung mindestens eines Lanthanoidsalzes, ausgewählt aus Lanthanoidnitrate, Lanthanoidcarbonate, Lanthanoidcarboxylate, bevorzugt Lanthanoidacetate, Lanthanoidsulphate, Lanthanoidoxide, besonders bevorzugt Pr₆O₁₁ und/oder Gd₂O₃, wobei die Lanthanoidion in den Lanthanoidoxiden oder Lanthanoidsalzen ausgewählt ist aus Praseodym, Gadolinium, Erbium, Neodym und für die Co-Dotierung mindestens zwei davon,
- ii) Bereitstellung eines Silikats, bevorzugt eines Silikatsalzes, besonders bevorzugt eines Alkalimetallsalzes des Silikats,
- iii) Bereitstellung mindestens eines Erdalkalimetallsalzes und mindestens eines Alkalimetallsalzes, bevorzugt eines Alkalimetallsilikats ausgewählt aus einem Lithiumsalz oder einer Lithiumverbindung und optional ausgewählt aus einem Natriumsalz und Kaliumsalz, vorzugsweise das Salz des Lithiumsalzes und ist ein Lithiumsilikat,
- iv) Bereitstellung mindestens eines Flussmittels aus der Gruppe der Ammoniumhalogenide, vorzugsweise Ammoniumchlorid, Alkalimetallhalogenide, vorzugsweise Natriumchlorid, Natriumfluorid, Natriumbromid, Lithiumfluorid oder Lithiumchlorid, Erdalkalimetallhalogenide, vorzugsweise Calciumchlorid oder Calciumfluorid, und Lanthanoidhalogenide, vorzugsweise Praseodymfluorid oder Praseodymchlorid,
- a) Mischen i), ii) iii) und iv) mittels Mahlens zur Erhaltung einer Mischung, oder
- b) Mischen i), ii) und iii) in einem organischen polaren oder unpolaren Lösemittel, das kein protisches Lösemittel ist, zur Erhaltung einer Mischung; die Mischung aus b) wird kalziniert (Schritt 1a) bei 600 bis 1000 °C zur Entfernung der organischen Komponente, bevorzugt wird die Kalzinierung bei 600 bis 1000 °C für mindestens 1 h durchgeführt, vorzugsweise mehr oder gleich 2 h, unter Standard (Luft)Atmosphäre zur Erhaltung einer kalzinierten Mischung,
- Kalzinieren der Mischung aus a) oder der kalzinierten Mischung aus b) in einem Kalzinierungsschritt, bevorzugt unter Luft bei einer Temperatur unterhalb der Schmelztemperatur des silikatbasierten Materials, wobei mindestens eine partielle Kristallisation stattfindet, bevorzugt in einem weiteren Kalzinierungsschritt (Schritt 1b) bei einer Temperatur von 50 bis 200 °C für mindestens 3h, vorzugsweise unter Luft, unterhalb der Schmelztemperatur des silikatbasierten Materials, um das silikatbasierte Material zu kristallisieren, bevorzugt bei einer Temperatur von 800 bis 900 °C, besonders bevorzugt bei ungefähr 850 °C, für mindestens 3 h, vorzugsweise für mindestens 12 h, vorzugweise unter Luft,
- in einem weiteren Kalzinierungsschritt bei steigender Temperatur, vorzugsweise über 800 °C und 50 bis 200 °C unterhalb des Schmelzpunktes (Schritt 2) des Materials, z.B. bei 850 °C für mindestens 3 h, besonders bevorzugt für mindestens 6 h, unter reduzierter Atmosphäre, dabei werden die Lanthanoide zu Ln³⁺ Ions reduziert,
- Erhalten eines silikatbasierten Lanthanoid-Ion dotierten Materials, bevorzugt nach Abkühlen des Materials.

Weitere detaillierte Ausführungsformen des Verfahrens können aus der EP 19202910.6 und PCT/EP2020/077798 entnommen werden, wobei für das erfindungsgemäße Verfahren ein Flussmittel zum Einsatz kommt.

Völlig überraschend konnte das bekannte Verfahren auf eine elegante Art und Weise modifiziert werden, was zudem zu optimierten up-conversion Leuchtstoffe mit außergewöhnlichen und unerwarteten Eigenschaften hinsichtlich der Partikelgrößenverteilung und Erhöhung der Emissionsintensität führt.

Vorzugsweise können 0,01 Gew.-% - 3,5 Gew.-%, bevorzugt 0,5 - 3,5 Gew.-%, besonders bevorzugt 1,0 - 3,5 Gew.-% Flussmittel, bezogen auf die Gesamtmenge der Edukte eingesetzt werden.

Es hat sich überraschend herausgestellt, dass die erfindungsgemäßen Leuchtstoffe, hergestellt angelehnt an die Lehre der EP 19202910.6 und PCT/EP2020/077798 die erforderlichen up-conversion Eigenschaften aufweisen, die für die antimikrobielle Wirkung verantwortlich sind. D. h. diese Leuchtstoffe können elektromagnetische Strahlung mit Wellenlängen oberhalb der UV-Strahlung, insbesondere sichtbares Licht oder Infrarotlicht, in elektromagnetische Strahlung mit geringerer Wellenlänge emittieren und zwar in dem Bereich, bei dem z.B. die DNA bzw. RNA der Mikroorganismen zerstört bzw. mutiert werden kann. Demnach sind diese Leuchtstoffe für die erfindungsgemäße Zusammensetzung sehr gut geeignet.

Es ist auch vorstellbar, den erfindungsgemäßen Leuchtstoff wie folgt herzustellen:
Als Ausgangsstoffe werden CaCO₃ (Fa. Alfa Aesar, 99.5%), Li₂CO₃ (Fa. Alfa Aesar, 99%), SiO₂ (Aerosil 200, Evonik), Pr₆O₁₁ (Treibacher, 99.99%), and Na₂CO₃ (Merck, 99.9%) sowie ein CaF₂ (Sigmaaldrich, 99.9 %) Flussmittel verwendet. Eine stöchiometrische Mischung dieser Verbindungen wird in Aceton für 30 Minuten vermischt. Nachdem das Aceton bei Raumtemperatur vollständig verdunstet ist, wird die Mischung in einem Korundtiegel überführt. Die Mischung wird zweimal kalziniert. Die erste Kalzinierung wird in einem Schmelzofen bei 850 °C für 12 h unter Luftzufuhr und die zweite Kalzinierung bei 850 °C für 6h unter 95/5 N₂/H₂ durchgeführt. Das Endprodukt wird in einer Achatreibschale anschließend gemahlen.

Es sei hier zu erwähnen, dass es möglich ist, durch eine nachträgliche Vermahlung des Leuchtstoffes gemäß der Lehre der EP 19202910.6 und PCT/EP2020/077798 einerseits die Homogenität der Partikelgröße und andererseits die gewünschte Partikelgröße zu erreichen. Jedoch würde hierbei der Energieeintrag höher liegen und der Vermahlungsprozess länger dauern bedingt durch deren Inhomogenität und Partikelgrößenverteilung nach der Herstellung.

Eine weitere Aufgabe der Erfindung ist die Auswahl filmbildender Polymere, die für die härtbare Zusammensetzung mit antimikrobieller Eigenschaft eingesetzt werden können. Grundsätzlich kommen alle aus dem Stand der Technik bekannten filmbildenden Polymere in Frage.

Vorzugsweise weist das filmbildende Polymer funktionelle Gruppen, vorzugsweise acide Wasserstoffe, auf, welche mit einem isocyanathaltigen Härter reaktiv sind und ggf. mit einem Katalysator katalysiert wird.

Vorteilhafterweise ist das filmbildende Polymer ausgewählt aus der Gruppe der hydroxyfunktionellen Acrylatpolymere, hydroxyfunktionellen Polyesterpolymere, und/oder hydroxyfunktionellen Polyetherpolymere, hydroxyfunktionellen Cellulosederivate, aminofunktionellen Asparticpolymer oder Polyesterpolymere, das mit einem isocyanathaltigen Härter reagiert.

Vorzugsweise ist das filmbildende Polymer resonanzarm.

Dem Fachmann sind die physikalischen Wechselwirkungen an der Oberfläche bekannt. Je nach Material und dessen Materialoberfläche treten bei Lichteinfall eine Vielzahl von Effekten an der Oberfläche auf. Das auftreffende Licht wird zum Teil absorbiert, ein Teil wird reflektiert und je nach Materialoberfläche auch gestreut. Licht kann auch erst absorbiert und dann wieder emittiert werden. Bei opaken, halbdurchlässigen oder lichtdurchlässigen Materialien kann das Licht auch durch den Körper durchdringen (Transmission). In einigen Fällen wird das Licht an der Oberfläche sogar polarisiert oder gebeugt. Manche Objekte können sogar Licht emittieren (beleuchtete Anzeigen, LED-Segmente, Bildschirme), in einer anderen Lichtfarbe fluoreszieren oder phosphoreszieren (nachleuchten). Resonanzarm bedeutet im Sinne dieser Erfindung, dass das filmbildende Polymer geringe Absorption, Reflexion, Remission und Streuung im UV-Bereich aufweist. Dagegen soll vorzugsweise die Transmission ausgeprägt sein.

Es konnte nämlich überraschend festgestellt werden, dass die erfindungsgemäße filmbildende Polymere, die resonanzarm sind, eine verbesserte antimikrobielle Wirkung aufweisen, bedingt dadurch, dass mehr elektromagnetische Strahlung mit geringerer Energie und höherer Wellenlänge im Bereich von 2000 nm bis 400 nm, insbesondere im Bereich von 800 nm bis 400 nm, transmittiert wird und resultierend daraus mehr elektromagnetische Strahlung mit höherer Energie und kürzerer Wellenlänge im Bereich von 400 nm bis 100 nm, bevorzugt im Bereich von 300 nm bis 200 nm, emittieren kann.

Es wurde festgestellt, dass je höher die Transmission ist, desto höher ist auch die Emission, die für die antimikrobielle Wirkung ausschlaggebend ist.

Vorzugsweise liegt die Transmission des filmbildenden Polymers bei mindestens 75%, bevorzugt mindestens 80% und besonders bevorzugt mindestens 85%, gemessen bei einer Wellenlänge von 260 nm.

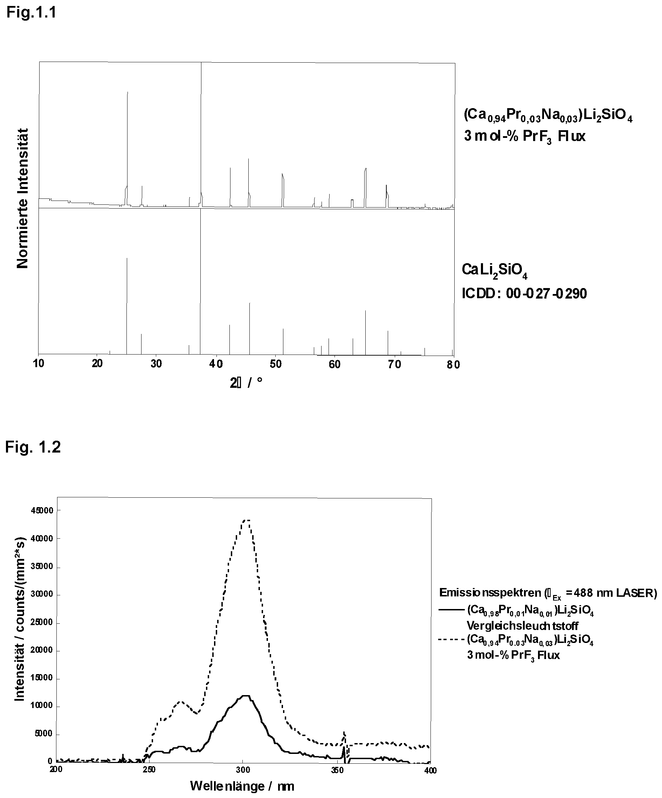

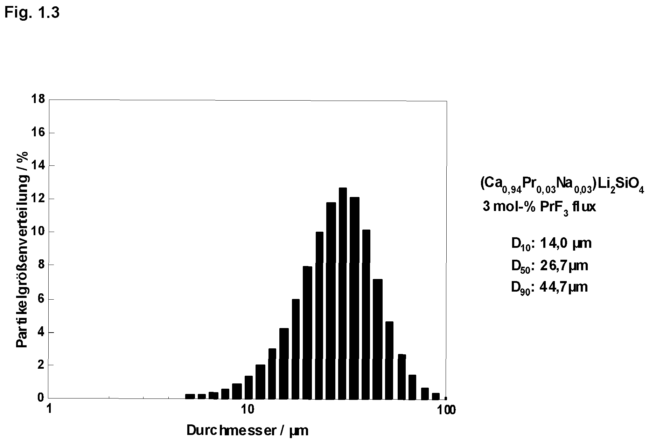

Vorzugsweise liegt die Transmission des filmbildenden Polymers bei mindestens 75%, bevorzugt mindestens 80% und besonders bevorzugt mindestens 85%, gemessen bei einer Wellenlänge von 500 nm.
Zur Verdeutlichung sei hier angemerkt, dass die Transmission bei einer anderen Wellenlänge definiert werden kann, siehe Abbildung 1. Für die vorliegende Erfindung wurden die Wellenlängen 260 nm beispielshaft für die emittierte Wellenlänge und 500 nm beispielhaft für die Anregungswellenlänge gewählt, die zum einen für die up-conversion und zum anderen signifikant für die antimikrobielle Wirkung verantwortlich sind.

Bei einer Transmission von 100% beispielsweise, gemessen bei einer Wellenlänge 260 nm, wird die gleiche Menge an Strahlung umgewandelt und emittiert, d. h. es gibt keine Verluste durch Absorption, Streuungen oder dergleichen. Bei einer Transmission von 80%, gemessen bei einer Wellenlänge 260 nm, transmittiert 20% nicht, vermutlich aufgrund von Absorption, Reflexion, Remission und/oder Streuung. Demzufolge kann nur 80% der Strahlung der Wellenlänge 260 nm emittiert werden.

Diese bedeutende Erkenntnis ist wichtig bei der Auswahl der filmbildende Polymere. Polymeren, die z.B. 0% Transmission aufweisen, eignen sich nicht für die erfindungsgemäße härtbare Zusammensetzung. Sie transmittieren keine elektromagnetische Strahlung mit geringerer Energie und höherer Wellenlänge und demzufolge können in der Zusammensetzung enthaltene Leuchtstoffe diese elektromagnetische Strahlung nicht in elektromagnetische Strahlung mit höherer Energie und kürzerer Wellenlänge umwandeln und emittieren, die für die antimikrobielle Wirkung erforderlich ist.

Vorzugsweise weist die erfindungsgemäße Zusammensetzung eine Transmission bei mindestens 75%, bevorzugt bei mindestens 80% und besonders bevorzugt bei mindestens 85% gemessen bei 260 nm beträgt.

Vorzugsweise weist die erfindungsgemäße Zusammensetzung eine Transmission bei mindestens 75%, bevorzugt bei mindestens 80% und besonders bevorzugt bei mindestens 85% gemessen bei 500 nm beträgt.

Die Transmissionskurven werden vorzugsweise mit einem "Specord 200 Plus" UV/VIS-Zweistrahlspektrometer der Firma Analytik Jena gemessen. Mit einem Holmiumoxidfilter erfolgt eine interne Wellenlängenkalibrierung. Die Proben werden mit monochromatisiertem Licht einer Deuterium- (UV-Bereich) oder einer Wolfram-Halogen-Lampe (sichtbarer Bereich) durchstrahlt. Die spektrale Bandbreite beträgt 1,4 nm. Das monochromatisierte Licht wird in einen Mess- und einen Referenzkanal aufgeteilt und ermöglicht das direkte Messen gegen eine Referenzprobe. Die durch die Probe transmittierende Strahlung wird von einer Photodiode detektiert und zu elektrischen Signalen verarbeitet.

Es ist vorstellbar, eine Zusammensetzung mit einer geringen Transmission von unter 70 % einzusetzen; sie wirken möglicherweise auch noch antimikrobiell, jedoch ist der Wirkungsgrad sehr moderat.

Vorzugsweise weisen die Leuchtstoffe eine durchschnittliche Partikelgröße von d50 von 0,1 - 50 µm, bevorzugt d50 = 0,1 - 25 µm, besonders bevorzugt d50 = 0,1 - 5 µm, gemessen nach ISO 13320:2020 und USP 429, beispielsweise mit einem Gerät der Fa. Horiba, LA-950 Laser Particle Size Analyzer, auf.

Um die Leuchtstoffe in der erfindungsgemäßen Zusammensetzung gut einzubinden und/oder zu stabilisieren, können vorzugsweise verschiedene Additive zugesetzt werden.

Bevorzugt sind die Additive ausgewählt aus der Gruppe der Dispergiermittel, Rheologiehilfsmittel, Verlaufsmittel, Netzmittel, Entschäumer und UV-Stabilisierungsmittel.

Es wurde überraschend festgestellt, dass durch etwaigen Zusatz an Additiven in die erfindungsgemäße Zusammensetzung die Transmission reduziert wird.

Demnach weist die erfindungsgemäße Zusammensetzung in einer weiteren Ausführungsform, in der Additive eingesetzt werden, vorzugsweise eine Transmission bei mindestens 70%, bevorzugt bei mindestens 75% und besonders bevorzugt bei mindestens 80% gemessen bei 260 nm beträgt.

Demnach weist die erfindungsgemäße Zusammensetzung in einer weiteren Ausführungsform, in der Additive eingesetzt werden, vorzugsweise eine Transmission bei mindestens 70%, bevorzugt bei mindestens 75% und besonders bevorzugt bei mindestens 80% gemessen bei 500 nm beträgt.

Vorzugsweise weist die erfindungsgemäße Zusammensetzung einen Härter auf, der ausgewählt ist aus der Gruppe der aliphatischen oder cycloaliphatischen Isocyanate.

Beispiele für isocyanathaltige Härter sind monomere Isocyanate, polymere Isocyanate und Isocyanat-Prepolymere. Polyisocyanate werden gegenüber monomeren Isocyanaten aufgrund ihrer geringeren Toxizität bevorzugt. Beispiele für Polyisocyanate sind Isocyanurate, Uretdione und Buirete basierend auf Diphenylmethan Diisocyanat (MDI), Toluol Diisocyanat (TDI), Hexamethylen Diisocyanate (HDI) and Isophoron Diisocyanat (IPDI). Beispiele für kommerziell erhältliche Produkte sind unter dem Markennamen DESMODUR^{®} von Covestro oder VESTANAT von der Evonik Industries. Bekannte Produkte sind DESMODUR^{®} N3400, DESMODUR^{®} N3300, DESMODUR^{®} N3600 DESMODUR^{®} N75, DESMODUR^{®} XP2580, DESMODUR^{®} Z4470, DESMODUR^{®} XP2565 and DESMODUR^{®} VL, von Covestro. Weitere Beispiele sind VESTANAT^{®} HAT 2500 LV, VESTANAT^{®} HB 2640 LV oder VESTANAT^{®} T 1890E von der Evonik Industries. Beispiele für Isocyanat-Prepolymere sind DESMODUR^{®} E XP 2863, DESMODUR^{®} XP 2599 oder DESMODUR^{®} XP 2406 von Covestro. Weitere dem Fachmann bekannte Isocyanat-Prepolymere können eingesetzt werden.

Es ist vorstellbar, Katalysatoren zur Aushärtung einzusetzen. Folgende Katalysatoren ausgewählt aus organischen Sn(IV)-, Sn(II)-, Zn-, Bi-Verbindungen oder tertiären Aminen können eingesetzt werden.
Bevorzugt werden Katalysatoren ausgewählt aus der Gruppe der Organozinnkatalysatoren, Titanate oder Zirkonate, metallorganische Verbindungen des Aluminiums, Eisens, Calciums, Magnesiums, Zinks oder Bismuths, Lewis-Säuren oder organische Säuren/Basen, lineare oder zyklische Amidine, Guanidine oder Amine oder eine Mischung daraus eingesetzt.

Vorzugseise werden als Härtungs-Katalysatoren organische Zinnverbindungen, wie z.B. Dibutylzinndilaurat, Dibutylzinndiacetylacetonat, Dibutylzinndiacetat, Dibutylzinndioctoat, oder Dioctylzinndilaurat, Dioctylzinndiacetylacetonat, Dioctylzinndiketanoat, Dioctylstannoxan, Dioctylzinndicarboxylat, Dioctylzinnoxid, bevorzugt Dioctylzinndiacetylacetonat, Dioctylzinndilaurat, Dioctylzinndiketanoat, Dioctylstannoxan, Dioctylzinndicarboxylat, Dioctylzinnoxid, besonders bevorzugt Dioctylzinndiacetylacetonat und Dioctylzinndilaurat eingesetzt. Des Weiteren können auch Zinksalze, wie Zinkoctoat, Zinkacetylacetonat und Zink-2-ethylcaproat, oder Tetraalkylammoniumverbindungen, wie N,N,N-Trimethyl-N-2-hydroxypropyl-ammonium-hydroxid, N,N,N-Trimethyl-N-2-hydroxypropylammonium-2-ethylhexanoat oder Cholin-2-ethylhexanoat verwendet werden. Bevorzugt ist die Verwendung von Zinkoctoat (Zink-2-ethylhexanoat) und der Tetraalkylammoniumverbindungen, besonders bevorzugt diejenige von Zinkoctoat. Weiter bevorzugt sind Bismutkatalysatoren, z.B. TIB Kat (TIB Mannheim) oder Borchi^{®}-Katalysatoren, Titanate, z.B. Titan(IV)isopropylat, Eisen(III)-Verbindungen, z.B. Eisen(III)-acetylacetonat, Aluminiumverbindungen, wie Aluminiumtriisopropylat, Aluminiumtri-secbutylat und andere Alkoholate sowie Aluminiumacetylacetonat, Calciumverbindungen, wie Calciumdinatriumethylendiamin-tetraacetat oder Calciumdiacetylacetonat, oder auch Amine, z.B. Triethylamin, Tributylamin, 1,4-Diazabicyclo[2,2,2]octan, 1,8-Diazabicyclo[5.4.0]undec-7-en, 1,5-Diazabicyclo[4.3.0]non-5-en, N,N-Bis-(N,N-dimethyl-2-aminoethyl)-methylamin, N,N-Dimethylcyclohexylamin, N,N-Dimethylphenylamin, N-Ethylmorpholin etc.. Auch organische oder anorganische Brönstedsäuren wie Essigsäure, Trifluoressigsäure, Methansulfonsäure, p-Toluolsulfonsäure oder Benzoylchlorid, Salzsäure, Phoshorsäure deren Mono- und/oder Diester, wie z.B. Butylphosphat, (Iso-)Propylphosphat, Dibutylphosphat etc., sind als Katalysatoren bevorzugt. Weiter bevorzugt sind Guanidin-Gruppen tragende organische und Silicium-organische Verbindungen. Selbstverständlich können auch Kombinationen mehrerer Katalysatoren eingesetzt werden. Darüber hinaus können auch photolatente Basen als Katalysatoren verwendet werden, wie sie in der WO 2005/100482 beschrieben sind.

Der Härtungskatalysator wird vorzugsweise in Mengen von 0,01 bis 5,0 Gew.-%, bevorzugt 0,05 bis 4,0 Gew.-% und besonders bevorzugt 0,1 bis 3 Gew.-% bezogen auf das Gesamtgewicht der härtbaren Zusammensetzung eingesetzt.

Bei filmbildenden Polymeren, die durch physikalische Trocknung aushärten, ist die Zugabe von reaktiven Härtern nicht erforderlich.

Die erfindungsgemäße Zusammensetzung können vorzugsweise in 1K-Beschichtungssysteme oder 2K- Beschichtungssysteme, in Melamin Einbrenn-Systeme, Raum- oder Hochtemperatursysteme eingesetzt werden.

Vorzugsweise weisen aus der erfindungsgemäßen Zusammensetzung hergestellte Beschichtungen eine antimikrobielle Wirkung gegen Bakterien, Hefen, Schimmelpilze, Algen, Parasiten und Viren auf.

Bevorzugt weisen die erfindungsgemäßen hergestellten Beschichtungen eine antimikrobielle Wirkung gegen
- Erreger nosokomialer Infektionen, vorzugsweise gegen *Enterococcus faecium, Staphylococcus aureus, Klebsiella pneumoniae, Acinetobacter baumannii, Pseudomonas aeruginosa, Escherichia coli, Enterobacter, Corynebacterium diphteria, Candida albicans,* Rotavirus, Bakteriophagen;
- Fakultativ pathogene Umweltorganismen, vorzugsweise gegen *Cryptosporidium parvum, Giardia lamblia,* Amöben *(Arcanthamoeba* spp., *Naegleria* spp.), *E. coli,* coliforme Bakterien, Fäkalstreptokokken, *Salmonella* spp., *Shigella* spp., *Leginonella* spec., *Pseudomonas aeruginosa, Mykobakteria* spp., enterale Viren (z.B. Polio- und Hepatitis-A Virus);
- Pathogene in Lebensmitteln, vorzugsweise gegen *Bacillus cereus, Campylobacter* spp., *Clostridium botulinum, Clostridium perfringens, Cronobacter* spp., *E. coli, Listeria monocytogenes, Salmonella* spp., *Staphylococcus aureus, Vibrio* spp., *Yersinia enterocolitica,* Bakteriophagen,
auf.

Es wurde festgestellt, dass die Einarbeitung der erfindungsgemäßen up-conversion Leuchtstoffe deutlich verbessert wurde.

Up-conversion Leuchtstoffe und Leuchtstoffe werden als Synonyme verwendet.

Ein weiterer Gegenstand ist die Verwendung der erfindungsgemäßen Zusammensetzung zur Herstellung von Dispersionen, Mahlgütern, Klebstoffen, Spachtelmassen, Putzen, Farben, Lacken oder Drucktinten, Inkjets, Anreibeharzen oder Pigmentkonzentraten.

Vorzugsweise ist die Verwendung der erfindungsgemäßen Zusammensetzung zur Herstellung von Beschichtungen mit anti-mikrobieller Eigenschaft.

Eine Beschichtung mit antimikrobieller Wirkung oder antimikrobieller Eigenschaft bedeutet hierbei, dass die Beschichtung eine antimikrobielle Oberfläche aufweist, die das Wachstum und die Vermehrung von Mikroorganismen begrenzt oder verhindert.

Es wurde erstaunlicherweise auch festgestellt, dass die erfindungsgemäßen Beschichtungen eine chemische und mechanische Beständigkeit aufweisen. Die chemische und mechanische Beständigkeit ist besonders bedeutsam, da antimikrobielle Beschichtungen häufig in Bereichen eingesetzt werden, die eine regelmäßige Desinfektion und weitere Hygienemaßnahmen erfordern.

Auch zur Erfindung gehört ein Verfahren zur Bildung einer anti-mikrobiellen Beschichtung auf einem Substrat, umfassend das Aufbringen einer härtbaren filmbildenden Zusammensetzung auf das Substrat, umfassend:
a. mindestens ein filmbildendes Polymer, das funktionelle Gruppen enthält, welche mit einem isocyanathaltigen Härter reaktiv sind, ggf. durch einen Katalysator katalysiert,
b. mindestens einen Leuchtstoff gemäß der Formel (II) und
c. einen Härter, enthaltend isocyanatfunktionelle Gruppen.

Vorzugsweise handelt es sich bei dem Substrat um Metall, mineralische Substrate (wie etwa Beton, Naturstein oder Glas), zellulosehaltige Untergründe, Holz sowie deren Hybride, formstabile Kunststoffe und/oder Duromere.

Unter dem Begriff "formstabile Kunststoffe" werden folgende, jedoch nicht abschließende, Polymere verstanden: Acrylnitril-Butadien-Styrol (ABS), Polyamide (PA), Polylactat (PLA), Polymethylmethacrylat (PMMA), Polycarbonat (PC), Polyethylenterephthalat (PET), Polystyrol (PS), Polyetheretherketon PEEK), Polyvinylchlorid (PVC). Polypropylen (PP), Polyethylen (PE).

Bevorzugt kann auf das Substrat vor der Auftragung der härtbaren filmbildenden Zusammensetzung eine Grundierzusammensetzung aufgetragen werden.

Vorzugsweise wird die erfindungsgemäße härtbare Zusammensetzung zur Beschichtung von Substraten in Hygieneeinrichtungen, Krankenhäusern und in der Lebensmittelindustrie eingesetzt.

Es können alle Bereiche des öffentlichen Raums, wie z.B. Schule, Altenheim, Großküche oder Kindergarten in Frage kommen.

Eine weitere Erfindung ist der Gegenstand, der mindestens teilweise, vorzugsweise vollständig, mit der erfindungsgemäßen härtbaren Zusammensetzung beschichtet ist.

Es sei hier anzumerken, dass die Begrifflichkeiten "antimikrobieller Effekt", antimikrobielle Wirksamkeit", "antimikrobielle Wirkung" und "antimikrobielle Eigenschaft" als Synonyme verwendet werden.

Es sei hier anzumerken, dass der erfindungsgemäße Gegenstand vorzugsweise auch ohne Freisetzung eines antimikrobiellen Wirkstoffs eine antimikrobielle Wirkung aufweisen kann, wenn die Beschichtung spezielle Leuchtstoffe, wie in den Ansprüchen beschrieben, enthalten. Auf diese Weise wird ein physikalischer Weg beschritten, auf dem die Mikroorganismen dann abgetötet werden. Daher fallen solche Materialien nicht unter die Biozid-Verordnung (Verordnung (EU), Nr. 528/2012 des Europäischen Parlaments und des Rates vom 22. Mai 2012 in der aktuellen Fassung von 2019).

Nachfolgend werden Beispiele aufgeführt, die dem Fachmann allein zur Erläuterung dieser Erfindung dienen und stellen keinerlei Beschränkung des beanspruchten Gegenstandes dar.

### Methoden

**Partikelgrößenverteilung** nach ISO 13320:2020 und USP 429, mit einem Gerät der Fa. Horiba, LA-950 Laser Particle Size Analyzer

**Qualitative Elementanalyse** mittels EDX mit Tabeltop 4000Plus von Hitachi, BSE-Detektor 15 kV, 1000fache Vergrößerung

**Pulver-XRDP:** Die Röntgenpulverdiffraktogramme der Proben wurden mit einem Panalytical X'Pert PRO MPD Diffractometer, das in der Bragg-Brentano Geometrie arbeitet, aufgneommen, wobei Cu-K_{α} Strahlung und ein Linescan CCD Detektor verwendet wurde. Die Integrationszeit betrug 20 s, während die Schrittweite 0.017° betrug.

**Die Emissionsspektren** wurden mit Hilfe eines Edinburgh Instruments FLS920 Spektrometers, das mit einem 488 nm continuous-wave OBIS Laser der Firma Coherent sowie einem Peltier gekühlten (-20 °C) single-photon counting photomultiplier der Firma Hamamatsu (R2658P) ausgerüstet ist. Kantenfilters wurden zur Unterdrückung der Reflexe zweiter und höherer Ordnung, welche durch die Monochromatoren verursacht werden, verwendet.

### I. Leuchtstoffe

### Beispiel 1 Erfindungsgemäßer Leuchtstoff (Ca_{0.94}Pr_{0.03}Na_{0.03})Li₂SiO₄ mit 3 mol-% PrF₃ als Flussmittel

2,8225 g (28,2 mmol) CaCO₃, 2,2167 g (30,0mmol) Li₂CO₃, 1,8025 g (30,0mmol) SiO₂, 0,0477 g (0,45 mmol) Na₂CO₃ und 0,1781 g (0,9 mmol) **PrF₃** wurden in einer Achatreibschale in Aceton vermischt. Diese Mischung wurde bei 850 °C für 12h in Luft zur Entfernung von organischen Bestandteilen kalziniert. Anschließend wird die Kalzinierung bei 850 °C für weitere 6 h in Formiergasatmosphäre (5% H₂/95% N₂) durchgeführt, was zum gewünschten Produkt führt. Der Leuchtstoff wurde für weitere Messungen entnommen.

Fig. 1.1 zeigt ein Röntgenpulverdiffraktogramm (X-ray diffraction pattern) des Leuchtstoffes aus Beispiel 1 (oberes Diagramm) im Vergleich zu einem nicht gefluxten Referenz-Leuchtstoff (unteres normiertes Röntgenpulverdiffraktogramm). Es konnte gezeigt werden, dass der gewünschte Leuchtstoff mit gewünschter Dotierung hergestellt wurde.

Fig. 1.2 zeigt ein Emissionsspektrum des Beispiels 1 (gestrichelte Linie) im Vergleich zu einem Vergleichs-Leuchtstoff, der nicht mit **PrF₃** gefluxt wurde. Die Herstellung des Vergleichs-Leuchtstoff (schwarze Linie) wurde analog Beispiel 1 durchgeführt, jedoch ohne PrF₃. Das Spektrum zeigt deutlich, dass die Intensität im gewünschten Wellenlängenbereich durch den Flussmittel-Zusatz gesteigert werden kann.

Fig. 1.3 zeigt eine Partikelgrößenverteilung des Beispiels 1. Es wurde die D₁₀, D₅₀ und D₉₀ gemessen. Folgende Werte konnten bestimmt werden:
D₁₀: 14,0 µm
D₅₀ : 26,7 µm
D₉₀: 44,7 µm

### Beispiel 2 Erfindungsgemäßer Leuchtstoff (Ca_{0.98}Pr_{0.01}Na_{0.01})Li₂SiO₄ mit 2,5 Gew.- % CaF₂ als Flussmittel

2,9426 g (29,4 mmol) CaCO₃, 2,2167 g (30,0 mmol) Li₂CO₃, 1,8025 g (30,0 mmol) SiO₂, 0,0511 g (0,05 mmol) Pr₆O₁₁, 0,0159 g (0,15 mmol) Na₂CO₃ und 0,1102 g (2,2029 mmol) CaF₂ wurden in einem Achatmörser in Aceton gemischt. Diese Mischung wurde bei 850 °C für 12 h in Luft zur Entfernung von organischen Bestandteilen kalziniert. Anschließend wird die Kalzinierung bei 850 °C für weitere 6 h in Formiergasatmosphäre (5% H₂/95% N₂) durchgeführt, was zum gewünschten Produkt führt.

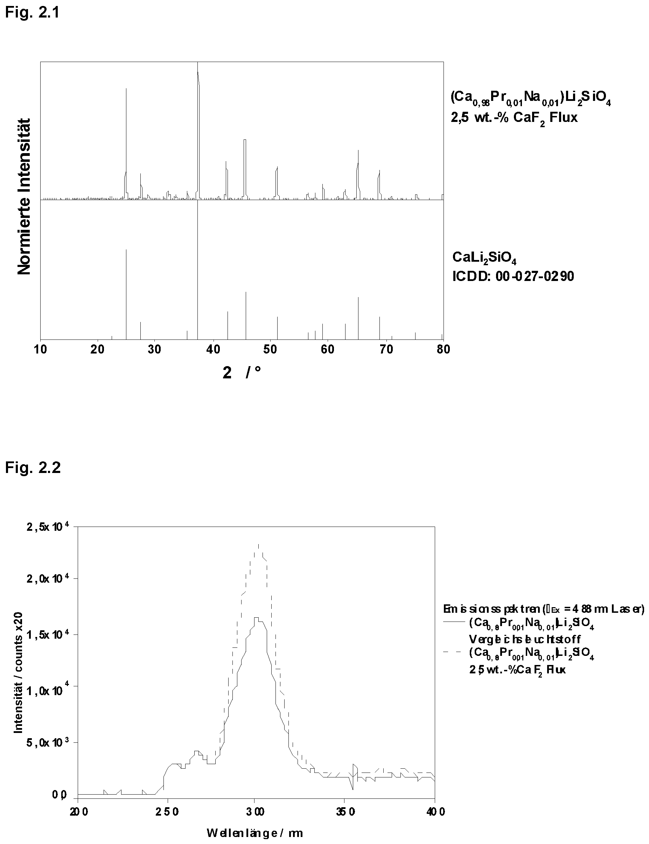

Fig. 2.1 zeigt ein Röntgenpulverdiffraktogramm (X-ray diffraction pattern) des Leuchtstoffes aus Beispiel 2 (oberes Diagramm) im Vergleich zu einem nicht gefluxten Referenz-Leuchtstoff (unteres normiertes Röntgendiagramm). Es konnte gezeigt werden, dass der gewünschte Leuchtstoff mit gewünschter Dotierung hergestellt wurde.

Fig. 2.2 zeigt ein Emissionsspektrum des Beispiels 2 (gestrichelte Linie) im Vergleich zu einem Vergleichs-Leuchtstoff, der nicht mit CaF₂ gefluxt wurde. Die Herstellung des Vergleichs-Leuchtstoff (schwarze Linie) wurde analog Beispiel 1 durchgeführt, jedoch ohne CaF₂. Das Spektrum zeigt deutlich, dass die Intensität im gewünschten Wellenlängenbereich durch den Flussmittel-Zusatz gesteigert werden kann.

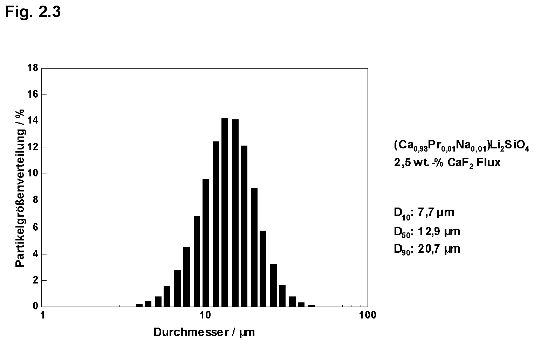

Fig. 2.3 zeigt eine Partikelgrößenverteilung des Beispiels 2 nach einem anschließenden Vermahlungsprozess. Die Partikelgröße konnte somit verkleinert werden. Es wurde die D₁₀, D₅₀ und D₉₀ gemessen. Folgende Werte konnten bestimmt werden:
D₁₀: 7,7 µm
D₅₀ : 12,9 µm
D₉₀ : 20,7 µm

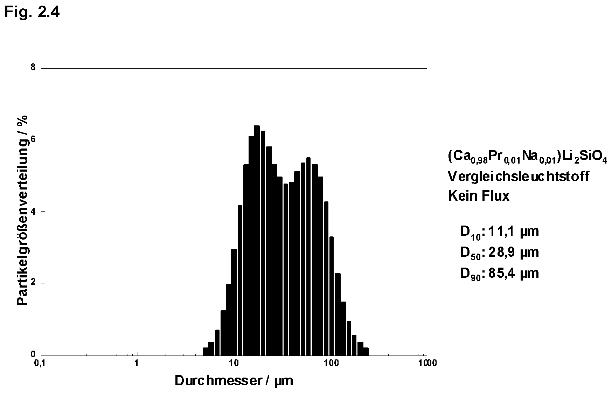

Fig. 2.4 zeigt eine Partikelgrößenverteilung des Vergleichs- Leuchtstoffes. Es wurde die D₁₀, D₅₀ und D₉₀ gemessen. Folgende Werte konnten bestimmt werden:
D₁₀ : 11,1 µm
D₅₀ : 28,9 µm
D₉₀ : 85,4 µm

Es konnte festgestellt werden, dass die Partikelgröße des erfindungsgemäßen Leuchtstoffes (Fig. 1.3) durch das Flussmittel homogener ist als die Partikelgröße des Vergleichs-Leuchtstoffes (Fig. 2.4). Somit kann der erfindungsgemäße Leuchtstoff leichter in die Lackmatrix eingearbeitet werden, was möglicherweise zu verbesserten Lackeigenschaften, wie das Erscheinungsbild der Lackoberfläche, beispielsweise der Glanz, die Haptik und der Touch führen können.

### Beispiel 3 Vergleichsbeispiel mit einem anderen Flussmittel, (Ca_{0.98}Pr_{0.01}Na_{0.01})Li₂SiO₄ mit 1 wt.-% H₃BO₃

2,9426 g (29,4 mmol) CaCO₃, 2,2167 g (30,0mmol) Li₂CO₃, 1,8025 g (30,0mmol) SiO₂, 0,0511 g (0,05mmol) Pr₆O₁₁, 0,0159 g (0,1500 mmol) Na₂CO₃ and 0,0441 g (0,7132 mmol) H₃BO₃ wurden in einer Achatschale in Aceton gemischt. Diese Mischung wurde bei 850 °C für 12 h in Luft zur Entfernung von organischen Bestandteilen kalziniert. Anschließend wird die Kalzinierung bei 850 °C für weitere 6 h in Formiergasatmosphäre (5% H₂/95% N₂) durchgeführt, was zum gewünschten Produkt führt. Der Leuchtstoff wurde für weitere Messungen entnommen.

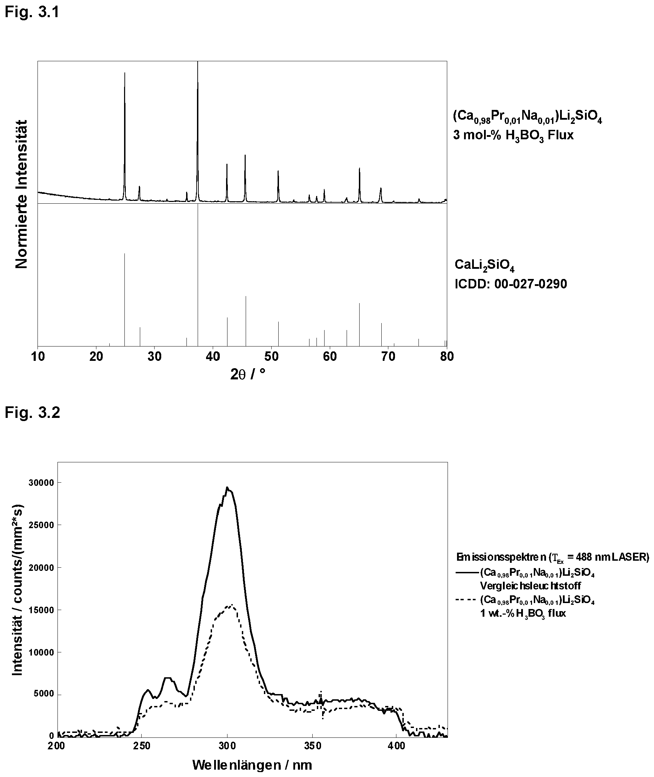

Fig. 3.1 zeigt ein Röntgenpulverdiffraktogramm (X-ray diffraction pattern) des Leuchtstoffes aus Beispiel 3 (oberes Diagramm) im Vergleich zu einem nicht gefluxten Referenz-Leuchtstoff (unteres normiertes Röntgendiagramm). Es konnte gezeigt werden, dass der gewünschte Leuchtstoff mit gewünschter Dotierung hergestellt wurde.

Fig. 3.2 zeigt ein Emissionsspektrum des Beispiels 2 (gestrichelte Linie) im Vergleich zu einem Vergleichs-Leuchtstoff, der nicht mit H₃BO₃ gefluxt wurde. Die Herstellung des Vergleichs-Leuchtstoff (schwarze Linie) wurde analog Beispiel 1 durchgeführt, jedoch ohne H₃BO₃. Das Spektrum zeigt deutlich, dass die Intensität des Leuchtstoffes nach Beispiel 3 im gewünschten Wellenlängenbereich durch den H₃BO₃-Zusatz reduziert wurde. Dieses Flussmittel scheint nicht geeignet zu sein.

Weitere Messungen wurden daher nicht mehr getätigt.

### Beispiel 4 Visueller Vergleich mittels Bildaufnahmen von gefluxten und ungefluxten Leuchtstoffen

Es wurden zwei Leuchtstoffe CaLi₂SiO₄:Pr³⁺,Na⁺(1%) analog hergestellt, wobei bei einem Leuchtstoff NaF als Flussmittel zugesetzt wurde.

2,9426 g (29,4 mmol) CaCO₃, 2,2167 g (30,0 mmol) Li₂CO₃, 1,8025 g (30,0 mmol) SiO₂, 0,0511g (0,05 mmol) Pr₆O₁₁, 0,0159 g (0,15 mmol) Na₂CO₃ und 0,1855 g (3,0 mmol) NaF wurden in einem Achatmörser in Aceton gemischt. Diese Mischung wurde bei 850 °C für 12 h in Luft zur Entfernung von organischen Bestandteilen kalziniert. Anschließend wird die Kalzinierung bei 850 °C für weitere 6 h in Formiergasatmosphäre (5% H₂/95% N₂) durchgeführt, was zum gewünschten Produkt führt.

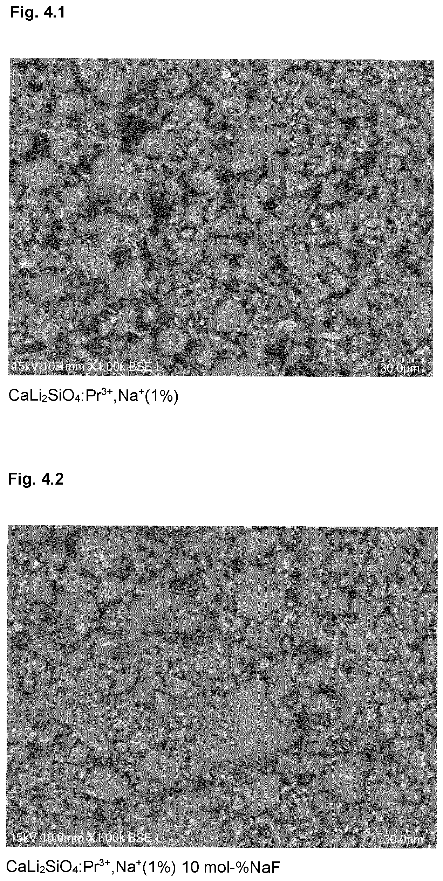

Fig. 4.1 zeigt eine Bildaufnahme des Leuchtstoffes CaLi₂SiO₄:Pr³⁺,Na⁺(1%), ohne Flussmittel. Die Aufnahme wurde mit einem BSE-Detektor bei 15 kV, bei einer 1000facher Vergrößerung durchgeführt.

Fig. 4.2 zeigt eine Bildaufnahme des Leuchtstoffes CaLi₂SiO₄:Pr³⁺,Na⁺(1%), mit NaF als Flussmittel. Die Aufnahme wurde mit einem BSE-Detektor bei 15 kV, bei einer 1000facher Vergrößerung durchgeführt.

Es ist ersichtlich, dass die Probe mit Flussmittel-Zusatz ein homogeneres Partikelbild ergibt.

### II. Anwendungsbeispiele

Es wurden analog die Beispiele aus der EP 21157055.1 durchgeführt. Methoden, Geräte und Materialien waren identisch mit denen aus der EP 21157055.1. Es wurde lediglich der erfindungsgemäße Leuchtstoff (gefluxt) ersetzt.

Es sei hier formal die Nummerierung der EP 21157055.1 aufgezählt, um eine Kopierung der besagten Textstellen und Tabellen zu vermeiden.

### 1. Auswahl der filmbildenden Polymere

1.1 Vorbereitung der Zusammensetzung ohne Leuchtstoffe und Additive
1.2 Beschichtung der Polymermatrices auf Quarzplatten
1.3 Transmissionsmessung

### 2. Auswahl der Additive

2.1 Transmissionsmessung
2.2 Überprüfung der Lackeigenschaften der Polymermatrices ohne Leuchtstoffe

### 3. Überprüfung der antimikrobiellen Wirksamkeit

3.1 Auswahl der Leuchtstoffe
- CaLi₂SiO₄:Pr³⁺,Na⁺(1%) gefluxt mit 10 mol% NaF, gemäß Beispiel 4
- CaLi₂SiO₄:Pr³⁺,Na⁺(1%) gefluxt mit 2,5 wt % CaF₂, gemäß Beispiel 2

### 3.2 Überprüfung der antimikrobiellen Wirksamkeit einer erfindungsgemäßen Zusammensetzung

Es sei hier anzumerken, dass die Begrifflichkeiten "antimikrobieller Effekt", antimikrobielle Wirksamkeit", "antimikrobielle Wirkung" und "antimikrobielle Eigenschaft" als Synonyme verwendet werden.

Zur Überprüfung der antimikrobiellen Wirksamkeit wurden die gefluxten Leuchtstoffe aus II. 3.1 jeweils in eine härtbare Zusammensetzung Z formuliert. Sie wurden gemäß den Angaben aus Tabelle 1 hergestellt. 50 g Glasperlen wurden zu der jeweiligen Zusammensetzung gegeben und für 5 min bei 2000 rpm im Speedmixer angerieben. Nach Abfiltrieren der Glasperlen wurde die jeweilige Zusammensetzung auf ein hochglanzgewälztes Alupanel aufgezogen und zu einem Film vernetzt, der eine Trockenschichtdicke von 50 µm aufwies. Auf dem Substrat ist nun eine Beschichtung, deren Beschichtungsoberfläche eine antimikrobielle Wirkung aufweisen soll, sowie deren Referenz ohne erwartete antimikrobielle Wirkung. Das Vergleichsbeispiel VZ enthält keine Leuchtstoffe.

**Tabelle 1: Rezepturen der härtbaren Zusammensetzungen für die Transfermethode**

| | **VZ [g]** | **Z2-1 [g]** | **Z2-2 [g]** | **Z4-1 [g]** | **Z4-2[g]** |
|---|---|---|---|---|---|
| CAB^{™} 381-2 | 6,82 | 6,82 | 6,82 | 6,82 | 6,82 |
| Butylacetat | 38,64 | 38,64 | 38,64 | 38,64 | 38,64 |
| CaLi₂SiO₄:Pr³⁺,Na⁺(1%) CaF₂ | | 0,90 | 1,33 | | |
| CaLi₂SiO₄:Pr³⁺,Na⁺(1%) NaF | | | | 0,90 | 1,33 |
| TIB Kat^{®} 218 | 0,05 | 0,05 | 0,05 | 0,05 | 0,05 |
| Desmodur^{®} N 3390 | 4,55 | 4,55 | 4,55 | 4,55 | 4,55 |

### 3.2.1 Transfermethode

Geprüft wurde an *Bacillus subtilits,* der im DVGW (Deutscher Verein des Gas- und Wasserfaches), Arbeitsblatt W 294 "UV-Geräte zur Desinfektion in der Wasserversorgung" zur biodosimetrischen Prüfung von UV-Anlagen eingesetzt wird. Als gram-positives sporenbildendes Bakterium ist er besonders unempfindlich gegenüber UV-Strahlung und damit als "worst case" zur Überprüfung der antimikrobiellen Wirkung von UV-Strahlung gut geeignet.
Als Testorganismus wurde *Bacillus subtilis* subsp. spizizenii (DSM 347, ATCC 6633) verwendet. Es wurde 1 ml einer B. *subtilis* Suspension mit einer finalen Konzentration von 10⁷ Zellen/mL auf einer sterilen CASO Agarplatte gleichmäßig verteilt, um eine konfluente Belegung des Nähragars zu gewährleisten. Die aufgetragene Bakteriensuspension wurde bei 300 ± 30 sec bei Raumtemperatur (22 ± 2 °C) auf dem Nähragar equilibriert. Die Bakteriensuspension wurden durch Verdünnungen von Vorkulturen des jeweiligen Bakterienstammes hergestellt. Verdünnt wurde in sterilem deionisierten Wasser. Die Vorkulturen der Testorganismen wurden in sterilisierter CASO Bouillon hergestellt. Die Vorkultur von B. *subtilis* wurde für 16 ± 1 h bei 30 °C unter konstantem Schütteln im Schüttelwasserbad inkubiert. Der Zelltiter der Vorkulturen wurde mikroskopisch mit einem Hämozytometer (Zählkammer nach Thoma) bestimmt.

Das Ziel der Transfermethode ist es, die antimikrobielle Wirkung der Beschichtungsoberfläche unter realitätsnahen Bedingungen auf einer trockenen unbelebten Oberfläche zu simulieren. Hierfür wurden die, wie oben beschrieben, erhaltenen Beschichtungen auf eine Größe von 2,5 cm x 4 cm zugeschnitten und auf eine konfluent mit B. *subtilis* inokulierte Nähragarplatte mit einem definierten Gewicht von 90 ± 1 g für 60 ± 5 sec gedrückt. Durch diesen Schritt wurden die Bakterien semi-trocken auf die Oberfläche der Beschichtung übertragen. Anschließend wurden die Substrate mit der beschichteten und inokulierten Seite nach oben in eine leere Petrischale gelegt und unter Beleuchtung bei Raumtemperatur für 0h, 1h, 2h, 3h, 5h inkubiert.

Zur Überprüfung der antimikrobiellen Wirkung durch den Effekt der Up-conversion wurden die Substrate mit der beschichteten und inokulierten Seite zusätzlich auch im Dunkeln bei Raumtemperatur für 0h, 1h, 2h, 3h, 5h inkubiert.

Alle Proben und die Referenz ohne UV-Upconverterpartikel wurden im Triplikat sowie mit und ohne Beleuchtung während der Inkubationszeit geprüft.

Die Detektion des antimikrobiellen Effekts nach der entsprechenden Inkubationszeit erfolgt über die Bestimmung der Kultivierbarkeit mit einen Abklatsch Test (Abbildung 1 der EP 21157055.1).

Für die Überprüfung der Kultivierbarkeit von *B*. *subtilis* wurden die Substrate mit der beschichteten und inokulierten Seite nach der Inkubationszeit von 0, 1, 2, 3, 5 h für 60 ± 5 sec mit einem definierten Gewicht von 90 ± 1 g auf eine sterile Nähragarplatte gedrückt. Der Nähragar wurde anschließend für 24 ± 1 h bei 30 °C statisch inkubiert. Die entstandenen bakteriellen Kolonien wurden visuell qualitativ ausgewertet.

### 3.2.2 Ergebnisse der Transfermethode

Ein wachstumsinhibierender Effekt auf die Bakterien kann bei der Transfermethode durch eine Abnahme der Kultivierbarkeit von *B*. *subtilis* überprüft werden.

Der erfindungsgemäße Leuchtstoff aus Beispiel 2 und 4 bewirken in der erfindungsgemäßen härtbaren Zusammensetzung Z2-1, Z2-2, Z4-1 und Z4-2 eine signifikante Verminderung der Kultivierbarkeit von *B*. *subtilis* im Vergleich zu VZ und den im Dunkeln inkubierten Proben (Tabelle 1). Die Kultivierbarkeit der adhärenten Bakterien auf der Beschichtungsoberfläche von den Vergleichsproben der härtbaren Zusammensetzung VZ zeigten eine deutliche Wachstumsinhibierung mit steigender Inkubationsdauer. Diese Verminderung konnte bereits nach 1 h Inkubation unter ständiger Beleuchtung gemessen werden. Die Abnahme der Kultivierbarkeit erhöht sich bis zu der Inkubationszeit von 5 h bei ständiger Beleuchtung. Die im Dunkeln inkubierten Zusammensetzungen zeigten über den Inkubationszeitraum von 5 h keine Verminderung der Kultivierbarkeit.

Durch die unveränderte Anzahl der kultivierbaren Bakterien über den Zeitraum von 5 h auf den in Dunkeln inkubierten Probenoberflächen kann gezeigt werden, dass der antimikrobielle Effekt des Leuchtstoffs nur im belichteten Zustand vorliegt.

Die härtbare Zusammensetzung VZ ohne die Zugabe von Up-Converter Partikeln zeigte weder im belichteten noch im abgedunkelten Zustand eine wachstumsinhibierende Wirkung auf *B*. *subtilis* (Tabelle 1).

Die polymeren Matrices zeigten zu dem keine genuine Kontamination. Dies wurde durch einen Abklatsch von Probenstücken ohne vorherigen Transfer von Bakterien auf diese überprüft.

Folglich kann bestimmt werden, dass die gefluxten Leuchtstoffe eine antimikrobielle Wirksamkeit in der erfindungsgemäßen Beschichtung, hergestellt aus der erfindungsgemäßen härtbaren Zusammensetzung, aufweisen.

**Tabelle 2: Antimikrobielle Wirksamkeit der härtbaren Zusammensetzungen**

| **Zusammensetzung** | **Antimikrobieller Effekt** | |
|---|---|---|
| | **Beleuchtet** | **Abgedunkelt** |
| VZ | Nein | Nein |
| Z2-1 | Ja | Nein |
| Z2-2 | Ja | Nein |
| Z4-1 | Ja | Nein |
| Z4-2 | Ja | Nein |

## Patentansprüche

1. Härtbare Zusammensetzung zur Herstellung von Beschichtungen mit anti-mikrobieller Eigenschaft enthaltend
- mindestens ein filmbildendes Polymer,
- optional mindestens ein Additiv,
- optional mindestens einen Härter,
- mindestens einen up-conversion Leuchtstoff der allgemeinen Formel (I)
A_{1-x-y-z}B*_{y}B₂SiO₄:Ln¹ₓ,Ln²_{z}, **I**
mit
x = 0,0001 - 0,0500;
z = 0,0000 oder z = 0,0001 bis 0,3000 mit der Maßgabe, dass gilt: y = x + z;
A ausgewählt aus der Gruppe bestehend aus Mg, Ca, Sr und Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
Ln¹ ausgewählt aus der Gruppe bestehend aus Praseodym (Pr), Erbium (Er) und Neodym (Nd);
Ln² ausgewählt aus Gadolinium (Gd),
wobei der Leuchtstoff mit mindestens einem halogenhaltigen Flussmittel hergestellt wurde.

2. Zusammensetzung nach Anspruch 1, **dadurch gekennzeichnet, dass** als Flussmittel mindestens ein Stoff aus der Gruppe der Ammoniumhalogenide, Alkalimetallhalogenide, Erdalkalimetallhalogenide und Lanthanoidhalogenide eingesetzt wird.

3. Zusammensetzung nach einem der vorgenannten Ansprüche, **dadurch gekennzeichnet, dass** es sich bei den Halogeniden um Fluoride, Bromide oder Chloride handelt.

4. Zusammensetzung nach einem der vorgenannten Ansprüche, **dadurch gekennzeichnet, dass** es sich bei den Alkalimetallen um Natrium oder Lithium handelt.

5. Zusammensetzung nach einem der vorgenannten Ansprüche, **dadurch gekennzeichnet, dass** es sich bei den Lanthanoiden um Praseodym handelt

6. Zusammensetzung nach einem der vorgenannten Ansprüche, **dadurch gekennzeichnet, dass** es sich bei den Erdalkalimetallen um Calcium handelt.

7. Zusammensetzung nach einem der vorgenannten Ansprüche, **dadurch gekennzeichnet, dass** der Leuchtstoff mit Praseodym dotiert ist.

8. Zusammensetzung nach einem der vorgenannten Ansprüche, **dadurch gekennzeichnet, dass** der Leuchtstoff mit Praseodym dotiert und mit Gadolinium co-dotiert ist.

9. Zusammensetzung nach einem der vorgenannten Ansprüche, **dadurch gekennzeichnet, dass** der Leuchtstoff eine erstarrte Schmelze aus kristallinen Silikaten oder aus kristallinen Silikaten dotiert mit Lanthanoid-Ionen, umfassend mindestens einen Alkalimetall Ion und mindestens einen Erdalkalimetall Ion, bevorzugt dass die kristalline Silikate mit Praseodym dotiert ist und optional co-dotiert mit Gadolinium, ist.

10. Zusammensetzung nach einem der vorgenannten Ansprüche, **dadurch gekennzeichnet, dass** der Leuchtstoff zumindest partiell kristallin ist.

11. Zusammensetzung nach einem der vorgenannten Ansprüche, **dadurch gekennzeichnet, dass** der Leuchtstoff ausgewählt ist aus Verbindungen der allgemeinen Formel (la)
A_{1-x-y-z}B*_{y}B₂SiO₄:Prₓ,Gd_{z}, Ia
mit A ausgewählt aus der Gruppe bestehend aus Mg, Ca, Sr, Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
x = 0,0001 - 0,0500;
z = 0,0000 oder z = 0,0001 bis 0,3000 mit der Maßgabe, dass gilt: y = x + z.

12. Zusammensetzung nach einem der vorgenannten Ansprüche, **dadurch gekennzeichnet, dass** der Leuchtstoff ausgewählt ist aus Verbindungen der allgemeinen Formel (II)
(Ca₁₋ₐSrₐ)_{1-2b}Ln_{b}Na_{b}Li₂SiO₄ II
wobei gilt:
Ln ist ausgewählt aus der Gruppe bestehend aus Praseodym, Gadolinium, Erbium, Neodym, vorzugsweise Praseodym;
a = 0,0000 bis 1,0000, vorzugsweise 0,0000 bis 0,1000, insbesondere 0,0000;
b = 0,0001 bis 1,0000, vorzugsweise 0,0001 bis 0,1000, insbesondere 0,0050 bis 0,0150.

13. Zusammensetzung nach einem der vorgenannten Ansprüche, **dadurch gekennzeichnet, dass** der Leuchtstoff ausgewählt ist aus Verbindungen der allgemeinen Formel (IIa)
Ca_{1-2b}Pr_{b}Na_{b}Li₂SiO₄ (IIa)
mit b = 0,0001 bis 1, vorzugsweise 0,0001 bis 0,1, insbesondere 0,005 bis 0,015.

14. Zusammensetzung nach einem der vorgenannten Ansprüche, **dadurch gekennzeichnet, dass** der Leuchtstoff Ca_{0,98}Pr_{0,01}Na_{0,01}Li₂SiO₄ oder Ca_{0,94}Pr_{0,03}Na_{0,03}Li₂SiO₄ ist.

15. Zusammensetzung nach einem der vorgenannten Ansprüche, **dadurch gekennzeichnet, dass** der Leuchtstoff ein Halogen, entsprechend dem Halogenid des Flussmittels, aufweist.

16. Zusammensetzung nach einem der vorgenannten Ansprüche, **dadurch gekennzeichnet, dass** der Leuchtstoff, der bei Bestrahlung mit elektromagnetischer Strahlung mit geringerer Energie und höherer Wellenlänge im Bereich von 2000 nm bis 400 nm, insbesondere im Bereich von 800 nm bis 400 nm, elektromagnetische Strahlung mit höherer Energie und kürzerer Wellenlänge im Bereich von 400 nm bis 100 nm, bevorzugt im Bereich von 300 nm bis 200 nm emittiert, wobei die Intensität des Emissionsmaximums der elektromagnetischen Strahlung mit höherer Energie und kürzerer Wellenlänge eine Intensität von mindestens 1 • 10³counts/(mm^{2*}s), bevorzugt höher als 1 • 10⁴ counts/(mm^{2*}s), besonders bevorzugt höher als 1 • 10⁵ counts/(mm^{2*}s).

17. Zusammensetzung nach einem der vorgenannten Ansprüche, **dadurch gekennzeichnet, dass** der Leuchtstoff gemäße Formel (II) XRPD Signale im Bereich von 23° 2Θ bis 27° 2Θ und von 34° 2Θ bis 39.5° 2Θ aufweist.

18. Zusammensetzung nach einem der vorgenannten Ansprüche, **dadurch gekennzeichnet, dass** das filmbildende Polymer funktionelle Gruppen, vorzugsweise acide Wasserstoffe, enthält, welche mit einem isocyanathaltigen Härter oder mit einem Katalysator reaktiv sind.

19. Zusammensetzung nach einem der vorgenannten Ansprüche, **dadurch gekennzeichnet, dass** das filmbildende Polymer aus der Gruppe der hydroxyfunktionellen Acrylatpolymere, hydroxyfunktionellen Polyesterpolymere, und/oder hydroxyfunktionellen Polyetherpolymere, hydroxyfunktionellen Cellulosederivate, aminofunktionellen Asparticpolymer oder Polyesterpolymere ausgewählt ist, das mit einem isocyanathaltigen Härter reagiert.

20. Zusammensetzung nach einem der vorstehenden Ansprüche, **dadurch gekennzeichnet, dass** das filmbildende Polymer resonanzarm ist.

21. Zusammensetzung nach einem der vorstehenden Ansprüche, **dadurch gekennzeichnet, dass** die Transmission des filmbildenden Polymers bei mindestens 75%, bevorzugt mindestens 80% und besonders bevorzugt mindestens 85%, mittels eines UV/VIS-Zweistrahlspektrometers liegt.

22. Zusammensetzung nach einem der vorstehenden Ansprüche, **dadurch gekennzeichnet, dass** die Transmission mindestens 70%, bevorzugt mindestens 75% und besonders bevorzugt mindestens 80%, mittels eines UV/VIS-Zweistrahlspektrometers beträgt.

23. Zusammensetzung nach einem der vorgenannten Ansprüche, **dadurch gekennzeichnet, dass** der Leuchtstoff eine durchschnittliche Partikelgröße von d50 = 0,1 - 50 µm, bevorzugt d50 = 0,1 - 25 µm, besonders bevorzugt d50 = 0,1 µm - 5 µm, gemessen nach ISO 13320:2020 und USP 429 aufweist.

24. Zusammensetzung nach einem der vorgenannten Ansprüche, **dadurch gekennzeichnet, dass** die Additive ausgewählt sind aus der Gruppe der Dispergiermittel, Rheologiehilfsmittel, Verlaufsmittel, Netzmittel, Entschäumer und UV-Stabilisierungsmittel.

25. Zusammensetzung nach einem der vorgenannten Ansprüche, **dadurch gekennzeichnet, dass** der Härter ausgewählt ist aus der Gruppe der aliphatischen oder cycloaliphatische Isocyanate.

26. Zusammensetzung nach einem der vorgenannten Ansprüche, **dadurch gekennzeichnet, dass** daraus hergestellte Beschichtungen eine antimikrobielle Wirkung gegen Bakterien, Hefen, Schimmelpilze, Algen, Parasiten und Viren aufweisen.

27. Zusammensetzung nach einem der vorgenannten Ansprüche, **dadurch gekennzeichnet, dass** daraus hergestellte Beschichtungen eine antimikrobielle Wirkung gegen
- Erreger nosokomialer Infektionen, vorzugsweise gegen *Enterococcus faecium, Staphylococcus aureus, Klebsiella pneumoniae, Acinetobacter baumannii, Pseudomonas aeruginosa, Escherichia coli, Enterobacter, Corynebacterium diphteria, Candida albicans,* Rotavirus, Bakteriophagen;
- Pathogene Umweltorganismen, vorzugsweise gegen *Cryptosporidium parvum, Giardia lamblia,* Amöben *(Arcanthamoeba* spp., *Naegleria* spp.), *E. coli,* coliforme Bakterien, Fäkalstreptokokken, *Salmonella* spp., *Shigella* spp., *Leginonella* spec., *Pseudomonas aeruginosa, Mykobakteria* spp., enterale Viren (z.B. Polio- und Hepatitis-A Virus);
- Pathogene in Lebensmitteln, vorzugsweise *gegenBacillus cereus, Campylobacter* spp., *Clostridium botulinum, Clostridium perfringens, Cronobacter* spp., *E. coli, Listeria monocytogenes, Salmonella* spp., *Staphylococcus aureus, Vibrio* spp., *Yersinia enterocolitica,* Bakteriophagen,
aufweisen.

28. Verwendung der Zusammensetzung nach einem der vorgenannten Ansprüche zur Herstellung von Dispersionen, Mahlgütern, Klebstoffen, Spachtelmassen, Putzen, Farben, Lacken oder Drucktinten, Inkjets, Anreibeharzen oder Pigmentkonzentraten.

29. Verwendung der Zusammensetzung nach einem der Ansprüche 1 bis 27 zur Herstellung von Beschichtungen mit anti-mikrobieller Eigenschaft.

30. Verwendung der Zusammensetzung nach einem der Ansprüche 1 bis 18 zur Beschichtung von Substraten in Hygieneeinrichtungen, Krankenhäusern und in der Lebensmittelindustrie.

31. Verfahren zur Bildung einer anti-mikrobiellen Beschichtung auf einem Substrat, umfassend das Aufbringen einer härtbaren filmbildenden Zusammensetzung auf das Substrat, umfassend:
(a) mindestens ein filmbildendes Polymer, das funktionelle Gruppen enthält, welche mit einem isocyanathaltigen Härter reaktiv sind, ggf. durch einen Katalysator katalysiert
(b) mindestens einen Leuchtstoff gemäß der Formel (II) und
(c) einen Härter, enthaltend isocyanatfunktionelle Gruppen.

32. Verfahren nach Anspruch 31, wobei das Substrat Metall, mineralische Substrate, zellulosehaltige Untergründe, Holz sowie deren Hybride formstabile Kunststoffe und/oder Duromere enthält.

33. Verfahren nach einem der Ansprüche 31 - 32, wobei eine Grundierzusammensetzung auf das Substrat vor der Auftragung der härtbaren filmbildenden Zusammensetzung aufgetragen wird.

34. Gegenstand, **dadurch gekennzeichnet, dass** er mindestens teilweise, vorzugsweise vollständig, mit der härtbaren Zusammensetzung nach einem der Ansprüche 1 bis 27 beschichtet ist.
